# EUROPÄISCHE PATENTSCHRIFT

(11) **EP 3 077 362 B1**
(45) Veröffentlichungstag und Bekanntmachung des Hinweises auf die Patenterteilung: **07.02.2018**
(21) Anmeldenummer: 14805914.0
(22) Anmeldetag: 02.12.2014
(51) Int. Cl.: C07C 67/05

(54) **VERFAHREN ZUR HERSTELLUNG VON VINYLACETAT MIT INHIBIERTER NEBENPRODUKTBILDUNG**
PROCESS FOR PRODUCING VINYL ACETATE WITH INHIBITED BY-PRODUCT FORMATION
PROCÉDÉ DE PRODUCTION D'ACÉTATE DE VINYLE METTANT EN OEUVRE UNE FORMATION DE PRODUITS SECONDAIRES INHIBÉE

(30) Priorität: 06.12.2013 DE 102013225114
(43) Veröffentlichungstag der Anmeldung: 12.10.2016
(73) Patentinhaber: Wacker Chemie AG, 81737 München (DE)
(72) Erfinder: DAFINGER, Willibald, 94133 Röhrnbach (DE); ECKERT, Marc, Calvert City, KY 42029 (US)
(74) Vertreter: Schuderer, Michael
(86) Internationale Anmeldenummer: PCT/EP2014/076210
(87) Internationale Veröffentlichungsnummer: WO 2015/082450

(56) Entgegenhaltungen:
- WO-A1-2010/149527

## Beschreibung

Die Erfindung betrifft ein Verfahren zur Herstellung von Vinylacetat in einem heterogen katalysierten, kontinuierlichen Gasphasenprozess durch Umsetzung von Ethylen mit Essigsäure und Sauerstoff und Aufarbeitung des Produktgasstromes, wobei die Nebenproduktbildung, aufgrund der Polymerisation von Vinylacetat, bei der Aufarbeitung des Produktgasstromes inhibiert wird.

Vinylacetat-Monomer (VAM) kann in einem kontinuierlichen Verfahren unter Rückführung des aufgereinigten Produktstromes hergestellt werden (Kreisgas-Prozess). Dabei reagiert in einem heterogen katalysierten Gasphasenprozess Ethylen mit Essigsäure und Sauerstoff an Katalysatoren, welche im Allgemeinen Palladium- und Alkalimetallsalze auf einem Trägermaterial enthalten und zusätzlich noch mit Gold, Rhodium oder Cadmium dotiert sein können. Bevorzugt wird ein Pd/Au-Katalysatorgemisch mit Kaliumacetat-Promotor eingesetzt.

Die Edukte Ethylen, Sauerstoff und Essigsäure werden in einer exothermen Reaktion (VAM: Δ_{B}H°₂₉₉ = - 176 kJ/mol), im Allgemeinen bei einem Überdruck von 7 bis 15 bar und, je nach Laufzeit des Katalysators, bei einer Temperatur von im Allgemeinen von 130°C bis 200°C, in einem Festbettröhrenreaktor, aber auch Fluidbettreaktoren, zu Vinylacetat umgesetzt:

C₂H₄ + CH₃COOH + ½ O₂ -> CH₃COOCH=CH₂ + H₂O

Hauptnebenreaktion ist dabei die Ethylen-Totaloxidation zu

CO₂: C₂H₄ + 3 O₂ -> 2 CO₂ + 2 H₂O

Der Ethylenumsatz liegt im Allgemeinen bei etwa 10 %, der Essigsäureumsatz bei 20 bis 30 % und der Sauerstoffumsatz bei bis zu 90 %.

Wegen des unvollständigen Umsatzes von Ethylen wird daher bei der Herstellung von Vinylacetat ein überwiegend aus Ethylen, Kohlendioxid, Ethan, Stickstoff und Sauerstoff bestehendes Gasgemisch im Kreis geführt. Der Gasstrom wird vor dem Festbettröhrenreaktor mit den Edukten Essigsäure, Ethylen und Sauerstoff versetzt und mittels mit Heizdampf betriebenen Wärmetauschern auf Reaktionstemperatur gebracht. Die Anreicherung des Kreisgases mit Essigsäure erfolgt üblicherweise mittels einem mit Heizdampf geheizten Essigsäuresättiger.

Nach der Reaktion werden im Allgemeinen die Reaktionsprodukte Vinylacetat und Wasser und nicht umgesetzte Essigsäure in einer sogenannten Vorentwässerungskolonne aus dem Kreisgas auskondensiert und der weiteren Aufarbeitung zugeführt. Nicht auskondensiertes Produkt, im Wesentlichen Ethylen, CO₂ und Vinylacetat, wird am Kopf der Vorentwässerungskolonne entnommen und in einem mit Essigsäure betriebenen Wäscher (Kreisgaswäscher) ausgewaschen. Das Kopfprodukt der Vorentwässerungskolonne (Kreisgas), oder zumindest eine Teilmenge davon, wird in einem CO₂-Wäscher vom gebildeten Kohlendioxid gereinigt. Das Kreisgas wird gegebenenfalls verdichtet, erneut mit den Edukten versetzt, und in den Reaktor zur Gasphasenoxidation geleitet.

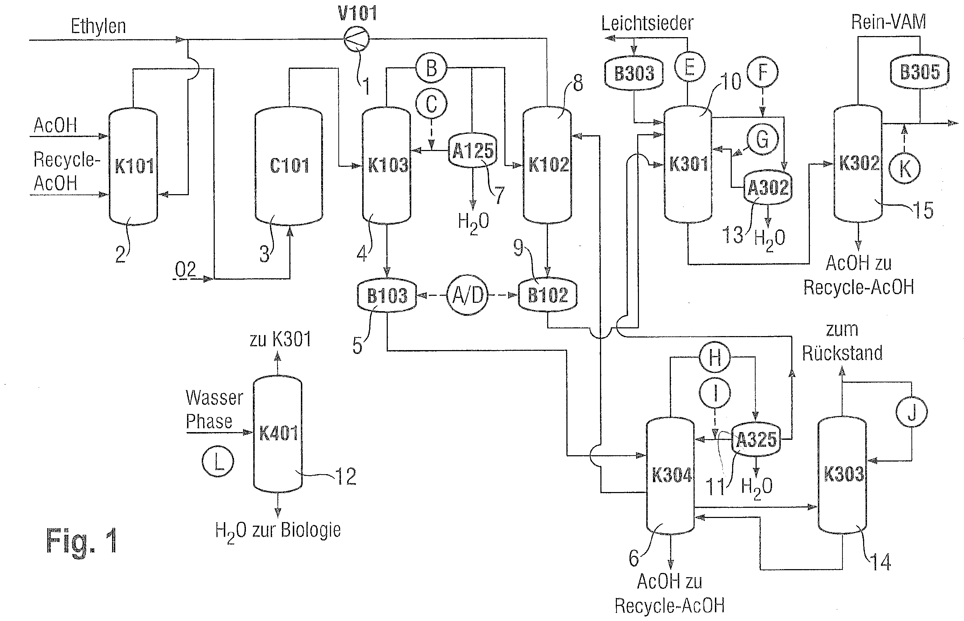

Zur weiteren Aufarbeitung des Kondensats aus der Vorentwässerungskolonne werden die auskondensierten Produkte Vinylacetat und Wasser sowie nicht umgesetzte Essigsäure in einem mehrstufigen, üblicherweise mit Heizdampf betriebenem, Destillationsprozess voneinander getrennt. Die üblichen Destillationsschritte zur Gewinnung des Vinylacetats und der Essigsäure sind Entwässerungskolonne, Azeotropkolonne, Rein-VAM-Kolonne, Rückstandsaufarbeitung, sowie Leichtsieder- und Hochsieder-Abtrennung (siehe Figur 1).

Bei der Aufarbeitung der Vinylacetat enthaltenden Fraktionen kommt es allerdings zur unerwünschten Polymerisation des Vinylacetat-Monomeren. Daraus resultieren Verschmutzungen und Belagsbildung (Fouling) in den einzelnen Kolonnen, Rohren und Behältern des Aufarbeitungsprozesses. Die dann notwendige Reinigung der Anlagenteile führt zu einem erheblichen Verlust an Produktionseffizienz. Im Extremfall muss sogar ein Produktionsstopp in Kauf genommen werden. Des Weiteren führt die VAM-Polymerisation zu Produktverlust, das heißt einer geringeren Ausbeute. Die Polymerisate erhöhen auch noch die Viskosität des ohnehin als zähflüssiger Teer abzutrennenden Nebenproduktgemisches. Die Verarbeitung und Abscheidung dieser Teere benötigt dann zunächst in den entsprechenden Apparateteilen eine höhere Temperatur und damit höhere Energiekosten, um sie in die entsprechende Entsorgungseinrichtungen zu verbringen. In der Folge können dann die Probleme in den einzelnen Anlagenteilen oder Kolonnen so massiv werden, dass eine Anlagenabstellung unumgänglich ist.

Etablierte Inhibitoren für die Polymerisation von Vinylacetat sind Chinone und Hydrochinone. Aus der DE-PS 1244161 ist der Einsatz von Chinonen zur Stabilisierung von Vinylacetat bei der Destillation bekannt. Obwohl die genannten Inhibitoren in relativ hohen Mengen von etwa 1000 bis 2000 ppm, bezogen auf die Menge an Vinylacetat, eingesetzt werden, kommt es immer wieder zu ungewollter Polymerisation, die zusätzliche Instandhaltungskosten erzeugt und teilweise zu einem immer reduzierteren Anlagendurchsatz führt. In der EP 1233937 B1 werden Kombination von Chinonen mit N-Oxyl-Verbindungen zur Inhibierung der Polymerisation von ungesättigten Monomeren, insbesondere Vinylaromaten, Acrylverbindungen und Dienen, empfohlen. Die Verwendung von gehinderten Phenolen wie 2,6-Di-tert-butyl-4-nonylphenol als Fouling-Inhibitor bei der Herstellung von Vinylacetat-Monomer in einem Kreisgasprozess ist aus der EP 567010 A1 bekannt. Der Inhibitor wird, gelöst in Essigsäure, dem Kreisgas vor dessen Komprimierung zur Rückführung in den Reaktor zugegeben.

Aus der WO 97/46504 A1 sind Stoffmischungen bekannt, welche vinylgruppenhaltige Verbindungen, und eine Kombination von 95,5 bis 99,5 % einer Nitroverbindung und 0,05 bis 4,5 % einer N-Oxylverbindung enthalten. Aus der WO 98/25872 A1 sind Stoffmischungen bekannt, enthaltend vinylgruppenhaltige Verbindungen, eine N-Oxyl-Verbindung eines sekundären Amins und eine Eisenverbindung. Die EP 791573 A1 bevorzugt den Einsatz von 2,2,6,6-**Te**tra**m**ethyl**p**iperidinyl**o**xyl (TEMPO), 4-Oxo-TEMPO oder von Estern des von 4-OH-TEMPO als Inhibitor für die Polymerisation von VAM. Von der Verwendung von Hydroxy- oder Aminofunktionellen N-Oxyl-Verbindungen wird abgeraten. In der WO 2007/045886 A1 werden hydrophobe TEMPO-Verbindungen, insbesondere Ether von 4-OH-TEMPO, als Polymerisationsinhibitoren für ethylenisch ungesättigte Verbindungen empfohlen. In der KR 10-2008-97529 werden Chinonverbindungen als Polymerisationsinhibitoren bei der Gasphasenoxidation empfohlen, während für die Aufarbeitung des Kreisgases TEMPO-Verbindungen empfohlen werden. In der WO 2012/058196 A1 werden ganz allgemein Radikalfänger (Scavenger) zur Behandlung von Vinylacetat-Zusammensetzungen aus der Gasphasenoxidation von Ethylen und Essigsäure empfohlen. Die WO 2010/149527 A1 beschreibt ein Verfahren zur Herstellung von Vinylacetat mittels Gasphasenoxidation von Ethylen und Essigsäure bei welchem N-Oxyl-Verbindungen während der Aufarbeitung des VAM-haltigen Produktstroms als Polymerisationsinhibitoren eingesetzt werden.

Es bestand daher die Aufgabe, die Aufarbeitung des Produktgasstromes aus der Vinylacetat-Herstellung so zu gestalten, dass die Nebenproduktbildung, aufgrund der Polymerisation des Vinylacetat-Monomeren, wirksam inhibiert wird.

Dazu geeignete N-Oxyl-Verbindungen sind im Allgemeinen 6-Ring-N-Oxyl-Verbindungen und im Handel erhältlich oder mit dem Fachmann bekannten Verfahren herstellbar. Die im Handel erhältlichen Formen sind Feststoffe oder deren Lösungen in Vinylacetat. Bei der Herstellung von 6-Ring-N-Oxyl-Verbindungen wird in der letzten Stufe eine sekundäre Amingruppe mittels Wasserstoffperoxid in die radikalische N-Oxyl-Gruppe umgewandelt. Die Reaktion erfolgt in Wasser und das Reaktionsprodukt fällt in wässriger Lösung, in hoher Konzentration von zumeist bis zu 80 Gew.-%, an. Bei den im Handel befindlichen Produkten wird das Wasser entfernt und der Feststoff gereinigt, beispielsweise mittels mehrfacher Umkristallisation, und die reine N-Oxyl-Verbindung gegebenenfalls in einem organischen Lösungsmittel gelöst. Bisher wurde nämlich davon ausgegangen, dass ohne Trocknung und Aufreinigung die Effektivität und Lagerbeständigkeit von N-Oxyl-Verbindungen unzureichend ist.

Überraschenderweise hat sich nun gezeigt, dass die Inhibitor-Aktivität der aus der letzten Synthesestufe hervorgehenden, hochkonzentrierten, wässerigen Lösung der N-Oxyl-Verbindungen, oder allgemein von wässerigen Lösungen von N-Oxyl-Verbindungen, sowohl lagerstabil als auch, was noch wesentlicher ist, genauso aktiv ist wie beim Einsatz von Feststoffen oder als umkristallisierter Feststoff, oder in Vinylacetat-Lösungen.

Gegenstand der Erfindung ist ein Verfahren zur Herstellung von Vinylacetat in einem heterogen katalysierten, kontinuierlichen Gasphasenprozess durch Umsetzung von Ethylen mit Essigsäure und Sauerstoff und anschließender Aufarbeitung des Produktgasstromes, wobei zur Inhibierung der Polymerisation von Vinylacetat, während der Aufarbeitung des Produktgasgemisches, ein oder mehrere N-Oxyl-Verbindungen, welche mindestens eine N-Oxyl-Radikal-Gruppe -N-O enthalten, als Inhibitoren zugesetzt werden, dadurch gekennzeichnet, dass die N-Oxyl-Verbindung in Form einer 50 bis 85 Gew.-%-igen wässerigen Lösung zugesetzt wird.

Bevorzugt werden als N-Oxyl-Verbindungen solche auf Basis von sekundären Aminen, in denen die N-Oxyl-Gruppe Bestandteil eines gesättigten oder ungesättigten sechsgliedrigen Ringes ist, beispielsweise Piperidin-1-Oxyl-Verbindungen, und bei denen die der N-Oxyl-Gruppe benachbarten C-Atome jeweils zwei C₁-bis C₄-Alkylgruppen, vorzugsweise Methylgruppen, tragen.

Besonders bevorzugt werden als 6-Ring-N-Oxyl-Verbindungen 2,2,6,6-Tetramethylpiperidinyloxyl (TEMPO), 4-Hydroxy-2,2,6,6-tetramethylpiperidinyloxyl (4-OH-TEMPO), 4-Oxo-2,2,6,6-tetramethylpiperidinyloxyl (4-Oxo-TEMPO) und 4-Ethanoyloxy-2,2,6,6-tetramethylpiperidinyloxyl. Am meisten bevorzugt werden 2,2,6,6-Tetramethylpiperidinyloxyl (TEMPO) und 4-Hydroxy-2,2,6,6-tetramethylpiperidinyloxyl (4-OH-TEMPO).

Die N-Oxyl-Verbindung wird im Allgemeinen als 50 bis 85 Gew.-%-ige, vorzugsweise 75 bis 85 Gew.-%-ige wässerige Lösung eingesetzt. Es wird die während der Synthese der N-Oxyl-Verbindungen anfallende, hoch konzentrierte, gegebenenfalls nicht weiter aus der letzten Stufe gereinigte, wässerige Lösung schon als solche als Inhibitor in dem erfindungsgemäßen Verfahren zur Herstellung von Vinylacetat eingesetzt. Die N-Oxyl-Verbindung kann auch in einer Wasser-Vinylacetat-Mischung eingesetzt werden. Vorzugsweise wird die N-Oxyl-Verbindung alleine und nicht in Kombination mit weiteren Additiven in wässriger Lösung oder gelöst in einer Wasser / Vinylacetat-Mischung eingesetzt.

Die für das Verfahren erforderliche Menge an N-Oxyl-Verbindung hängt zum Einen von der Konstruktion und Kapazität der jeweiligen Anlage ab. Die erforderliche Menge kann durch Monitoring und Analyse der Stoffströme in der Anlage, das heißt mittels quantitativer Bestimmung der Gesamtpolymerbildung oder Polymerbildung in einzelnen Anlageteile festgestellt werden. Die Einsatzmenge hängt auch davon ab, ob mit Einsatz der N-Oxyl-Verbindung eine prophylaktische Behandlung zur Laufzeitverlängerung der gesamten Anlage angestrebt wird, oder der Polymeranfall in einzelnen Anlageteilen inhibiert werden soll, um dort den Behandlungsaufwand für die Reinigung zu reduzieren.

Im Allgemeinen sind Einsatzmengen von 50 bis 500 ppm, vorzugsweise 50 bis 200 ppm an N-Oxyl-Verbindung, jeweils pro Tonne Vinylacetat im Produktgasstrom, für die gesamte Anlage ausreichend. Diese Einsatzmenge kann an einer oder verteilt über mehrere Stellen der Anlage zur Aufarbeitung des Produktgasstromes zugegeben werden, das heißt in einer oder mehreren Kolonnen, Leitungen oder Vorratsbehälter.

Bevorzugte Stellen A bis L zur Zugabe des Inhibitors sind in Figur 1 aufgezeigt.

In Figur 1 ist ein vereinfachtes Schema für die Herstellung von Vinylacetat in einem Gasphasenprozess und anschließender Aufarbeitung des Produktgasstromes angegeben.

Das mit dem Kreisgasverdichter V-101 (1) verdichtete Kreisgas wird mit Frischethylen, das den bei der Reaktion verbrauchten Ethylenanteil ersetzt, angereichert und dem Essigsäuresättiger K-101 (2) zugeführt. Die in der Reaktion umgesetzte Essigsäure wird im Essigsäuresättiger (2) durch Einspeisung von Frischessigsäure ersetzt. Die Hochsieder und andere Nebenprodukte, wie beispielsweise alle rückgeführten Polymere und nicht verbrauchte Inhibitoren werden am Sumpf des Essigsäuresättigers (2) abgezogen, von Rest-Essigsäure in der Essigsäureaufarbeitung befreit und die verbleibenden Rückstände entsorgt.

Da in der Reaktion keine vollständige Umsetzung der Essigsäure erfolgt, wird in den nachfolgenden Destillationen (beispielsweise in (6) und (15)) diese Essigsäure jeweils am Sumpf abgezogen und dem Reycycle-Essigsäure-Tank zugeführt.

Dem den Essigsäuresättiger K-101 (2) verlassenden, mit Essigsäure beladenen Kreisgas wird vor Eintritt in das Reaktorsystem über eine Düse Sauerstoff zugegeben. Anschließend wird das Kreisgas mit einem Kreisgasdruck von 7 bis 15 bar abs. dem Festbettröhrenreaktor C-101 (3) zugeführt, welcher mit einem Pd/Au-Katalysatorgemisch mit Kaliumacetat-Promotor beladen ist, und bei einer Temperatur von 130 bis 200°C betrieben wird.

Der den Festbettröhrenreaktor C-101 (3) verlassende Gasstrom wird dem unteren Teil der Vorentwässerungs-Kolonne K-103 (4) zugeführt. Ein erstes Kondensat (Vinylacetat, Wasser und nicht umgesetzte Essigsäure) aus dieser Kolonne wird in den Roh-VAM-Behälter B-103 (5) geleitet. Der Roh-VAM-Behälter B-103 (5) ist Stelle A an der die Inhibitorzudosierung erfolgen kann.

Das Roh-VAM aus dem Roh-VAM-Behälter B-103 (5) wird danach in die Azeotrop-Kolonne K-304 (6) gepumpt. Der Hauptgas-Strom aus der Vorentwässerungs-Kolonne K-103 (4) kann vor der nachfolgenden Kondensation im Kreisgaswäscher K-102 (8) inhibiert werden (Stelle B). Zur Inhibierung des Rücklaufs aus dem Phasentrenner A-125 (7) kann die N-Oxyl-Verbindung auf dem Weg zur Vorentwässerungskolonne K-103 (4) zugegeben werden (Stelle C) .

Der nicht kondensierte Bestandteil der Kopfbrüden der Vorentwässerungskolonne (4), im wesentlichen Ethylen, CO₂ und Vinylacetat wird zum Kreisgaswäscher K-102 (8) abgegeben. Die nicht kondensierten VAM-Anteile werden im mit Essigsäure betriebenen Kreisgaswäscher K-102 (8) absorbiert. Die zur Kreisgaswäsche erforderliche Absorptions-AcOH kann von der Azeotrop-Kolonne K-304 (6) zugeführt werden. Das nun VAM-freie Kreisgas wird über den Kreisgas-Verdichter V-101 (1) und dem Essigsättiger K-101 (2) wieder der Reaktion im Reaktor C-101 (3) zugeführt. Das Sumpfprodukt aus dem Kreisgaswäscher K-102 (8) wird in den Roh-VAM-Behälter B-102 (9) geleitet und von dort in die Entwässerungs-Kolonne K-301 (10). In den Roh-VAM-Behälter B-102 (9) und/oder in den ROH-VAM-Behälter B-103 (5) kann ebenfalls Inhibitor zugegeben werden (Stelle D).

Vorzugsweise wird in die Entwässerungs-Kolonne K-301 (10) im Allgemeinen noch ein zweiter Roh-VAM-Strom geleitet: Das Roh-VAM aus dem Roh-VAM-Behälter B-103 (5) (= Kondensat aus Vorentwässerung (4)). Dieses wird zunächst in der Azeotrop-Kolonne K-304 (6) destilliert. Das Kopfprodukt dieser Destillation in der Azeotrop-Kolonne K-304 (6), im Wesentlichen Vinylacetat und Wasser, wird zur Wasser-Abtrennung in den Phasentrenner A-325 (11) überführt, und kann zwischen Azeotrop-Kolonne (6) und Phasentrenner (11) mit Inhibitor versetzt werden (Stelle H).

Der Großteil der organischen Phase (im Wesentlichen VAM) aus dem Phasentrenner A-325 (11) wird als Rücklauf wieder in die Azeotrop-Kolonne K-304 (6) zurückgepumpt und kann vorher inhibiert werden (Stelle I). Der verbleibende Teil der organischen Phase wird zur Entwässerungs-Kolonne K-301 (10) verbracht.

Die wässerige Phase des Phasentrenners A-325 (11) wird zur Abwasser-Kolonne K-401 (12) gefördert, in der alle wässerigen Phasen aus den Phasentrennern A-125 (7), Phasentrenner A-302 (13) und Phasentrenner A-325 (11) der gesamten Destillation aufgearbeitet werden. Das wässerige Sumpfprodukt der AbwasserKolonne (12) wird entsorgt, das Kopfprodukt in die Entwässerungskolonne K-301 (10) zurückgeführt. Zur Inhibierung kann der Inhibitor am Einlass der wässerigen Phasen in die Abwasser-Kolonne K-401 (12) zugeführt werden (Stelle L).

Ein Seitenabzug der Azeotrop-Kolonne K-304 (6) kann zur Entfernung des Ethylacetats zur Ethylacetat-Kolonne K-303 (14) geleitet werden. Im Rücklauf dieser Ethylacetat-Kolonne K-303 (14) kann ebenfalls Inhibitor zudosiert werden (Stelle J).

Vorzugsweise wird in der Entwässerungskolonne K-301 (10) im Wesentlichen das Sumpfprodukt aus dem Kreisgaswäscher K-102 (8), welches im Wesentlichen Wasser, Vinylacetat, Essigsäure und Leichtsieder (vor allem Acetaldehyd) enthält, aufgetrennt: Die Leichtsieder und Wasser werden von Vinylacetat und Essigsäure abgetrennt. Dabei wird über den Kopf der Entwässerungskolonne K-301 (10) der hier durch Vinylacetat-Hydrolyse gebildete Acetaldehyd abgetrennt, nachfolgend kondensiert und zur weiteren Aufarbeitung gepumpt. An dieser Stelle kann Inhibitor zugegeben werden (Stelle E).

Über einen Zwischenboden der Entwässerungskolonne K-301 (10) kann der wasserhaltige Seitenabzug zum Phasentrenner A-302 (13) geleitet werden. An dieser Leitung kann Inhibitor zugegeben werden (Stelle F). Die organische Phase wird gegebenenfalls inhibiert (Stelle G) und dann, an gegebenenfalls mehreren Stellen, als Rücklauf der Entwässerungskolonne K-301 (10) wieder zugeführt.

Das Sumpfprodukt der Entwässerungskolonne K-301 (10), Vinylacetat und Essigsäure, wird zur Rein-VAM-Kolonne K-302 (15) geleitet. Über Kopf wird Rein-VAM abgetrennt und teilweise als Rücklauf zurückgeführt. An diesem Rücklauf kann ebenfalls Inhibitor zugegeben werden (Stelle K). Das Sumpfprodukt, im Wesentlichen Essigsäure, wird der Essigsäure-Aufarbeitung zugeführt.

In diesem Schema in Figur 1 werden die CO₂-Wäsche und die Essigsäure-Aufarbeitung nicht beschrieben, da in den zugehörigen Prozessschritten keine oder nur geringe Mengen an Vinylacetat zugegen sind, und daher im Allgemeinen dort keine Inhibitorzugabe erforderlich ist.

Überraschenderweise konnte gezeigt werden, dass der Einsatz einer wässerigen Lösung, direkt aus der Synthese der N-Oxyl-Verbindung kommend, gegenüber dem Einsatz von Hydrochinon (siehe Vergleichsbeispiel 1) deutliche Vorteile hatte.

Die nachfolgenden Beispiele dienen der weiteren Erläuterung der Erfindung:
In einer Anlage gemäß Figur 1, welche unter den oben genannten Bedingungen (Kreisgasdruck 7 bis 15 bar abs., Reaktionstemperatur 130 bis 200°C) mit einer Gas Hourly Space Velocity (GHSV) von ca. 3000 bis 4000 [1/h] und einer Space Time Yield (STY) von 600 bis 1200 (kg VAM/ m³ Kat x h) betrieben wurde, wurde an den Stellen A bis L jeweils Inhibitor zugegeben.

Aus den Sümpfen folgender Kolonnen wurden Proben zur Analyse entnommen und die Polymerbildung in den Kolonnen quantitativ analysiert:
Azeotrop-Kolonne K-304 (6); Entwässerungskolonne K-301 (10) und Rein-VAM-Kolonne K-302 (15).

### Vergleichsbeispiel 1:

Es wurde zunächst über einen Zeitraum von einem Monat an den Stellen A bis L insgesamt 1300 bis 2100 ppm Hydrochinon pro Tonne VAM zugegeben.

Nach einem Monat wurden folgende Polymerbildungsraten in den Kolonnen gemessen:

| | |
|---|---|
| Entwässerungskolonne K-301 (11): | 20 - 50 kg/h |
| Rein-VAM-Kolonne K-302 (16): | 5 - 8 kg/h |
| Azeotrop-Kolonne K-304 (9): | 15 - 20 kg/h |

### Beispiel 1:

Nach Ablauf von Vergleichsbeispiel 1 wurden über einen Zeitraum von einem weiteren Monat an den Stellen A bis L insgesamt 50 bis 200 ppm 4-Hydroxy-2,2,6,6-tetramethylpiperidin-1-oxyl (4-OH-TEMPO) pro Tonne VAM, jeweils als 75 Gew.-%-ige wässrige Lösung, zugegeben.

Nach Ablauf des Monats der Inhibierung mit der wässrigen OH-TEMPO-Lösung wurden folgende Polymerbildungsraten in den Kolonnen gemessen:

| | |
|---|---|
| Entwässerungskolonne K-301 (11): | 10 - 20 kg/h |
| Rein-VAM-Kolonne K-302 (16): | 1 - 3 kg/h |
| Azeotrop-Kolonne K-304 (9): | 8 - 10 kg/h |

### Beispiel 2:

Nach Ablauf von Beispiel 1 wurden über einen Zeitraum von einem weiteren Monat an den Stellen A bis L insgesamt 50 bis 200 ppm 2,2,6,6-Tetramethylpiperidin-1-oxyl (TEMPO) pro Tonne VAM, jeweils als 75 Gew.-%-ige wässrige Lösung, zugegeben.

Nach Ablauf des Monats der Inhibierung mit der wässrigen TEMPO-Lösung wurden folgende Polymerbildungsraten in den Kolonnen gemessen:

| | |
|---|---|
| Entwässerungskolonne K-301 (11): | 11 - 22 kg/h |
| Rein-VAM-Kolonne K-302 (16): | 2 - 4 kg/h |
| Azeotrop-Kolonne K-304 (9): | 10 - 12 kg/h |

Trotz der wesentlich geringeren Einsatzmengen an TEMPO bzw. 4-OH-TEMPO und deren Einsatz als hochkonzentrierte, wässrige Lösung wurde im Vergleich zur einmonatigen Inhibierung mit Hydrochinon eine wesentlich effektivere Inhibierung der Polymerisatbildung erreicht.

## Patentansprüche

1. Verfahren zur Herstellung von Vinylacetat in einem heterogen katalysierten, kontinuierlichen Gasphasenprozess durch Umsetzung von Ethylen mit Essigsäure und Sauerstoff und anschließender Aufarbeitung des Produktgasstromes, wobei zur Inhibierung der Polymerisation von Vinylacetat, während der Aufarbeitung des Produktgasgemisches, ein oder mehrere N-Oxyl-Verbindungen, welche mindestens eine N-Oxyl-Radikal-Gruppe -N-O· enthalten, als Inhibitoren zugesetzt werden, **dadurch gekennzeichnet, dass** die N-Oxyl-Verbindung in Form einer 50 bis 85 Gew.-%-igen wässerigen Lösung zugesetzt wird, wobei als wässrige Lösung der N-Oxyl-Verbindung die bei der Synthese der N-Oxyl-Verbindung anfallende, wässrige Lösung eingesetzt wird.

2. Verfahren nach Anspruch 1, **dadurch gekennzeichnet, dass** als N-Oxyl-Verbindungen solche zugesetzt werden, aus der Gruppe der sekundären Aminen, in denen die N-Oxyl-Gruppe Bestandteil eines gesättigten oder ungesättigten sechsgliedrigen Ringes ist, und bei denen die der N-Oxyl-Gruppe benachbarten C-Atome jeweils zwei C1- bis C4-Alkylgruppen tragen.

3. Verfahren nach Anspruch 1 oder 2, **dadurch gekennzeichnet, dass** als N-Oxyl-Verbindung 2,2,6,6-Tetramethylpiperidinyloxyl, 4-Hydroxy-2,2,6,6-tetramethylpiperidinyloxyl, 4-Oxo-2,2,6,6-tetramethylpiperidinyloxyl und/oder 4-Ethanoyloxy-2,2,6,6-tetramethylpiperidinyloxyl zugesetzt wird.

4. Verfahren nach Anspruch 1 bis 3, **dadurch gekennzeichnet, dass** die N-Oxyl-Verbindungen in einer Wasser-Vinylacetat-Mischung eingesetzt werden.

5. Verfahren nach Anspruch 1 bis 4, **dadurch gekennzeichnet, dass** pro Tonne Vinylacetat im Produktgasstrom 50 bis 500 ppm an N-Oxyl-Verbindung zugesetzt werden.

6. Verfahren nach Anspruch 1 bis 5, **dadurch gekennzeichnet, dass** die N-Oxyl-Verbindung an einer oder verteilt über mehrere Stellen der Anlage zur Aufarbeitung des Produktgasstromes zugesetzt wird.

7. Verfahren nach Anspruch 6, **dadurch gekennzeichnet, dass** die N-Oxyl-Verbindung
dem Kondensat aus der Vorentwässerungskolonne und/oder
dem Gasstrom aus der Vorentwässerungskolonne und/oder
dem Rücklauf zur Vorentwässerungskolonne und/oder
dem Sumpfprodukt aus dem Kreisgaswäscher und/oder
dem Kopfprodukt der Azeotropkolonne und/oder
dem Rücklauf in die Azeotropkolonne und/oder
dem Einlass in die Abwasserkolonne und/oder
dem Rücklauf in die Ethylacetat-Kolonne und/oder
dem Kopfprodukt der Entwässerungskolonne und/oder
dem Seitenabzug der Entwässerungskolonne und/oder
dem Rücklauf in die Entwäserungskolonne und/oder
dem Kopfprodukt der Reinvinylacetat-Kolonne
zugesetzt wird.

## Claims

1. Process for preparing vinyl acetate in a heterogeneously catalyzed, continuous gas phase process by reacting ethylene with acetic acid and oxygen and subsequently working up the product gas stream, with inhibition of the polymerization of vinyl acetate during the workup of the product gas mixture by addition of one or more N-oxyl compounds which contain at least one N-oxyl radical group -N-O· as inhibitors, **characterized in that** the N-oxyl compound is added as a 50 to 85% by weight strength aqueous solution, wherein the aqueous solution of the N-oxyl compound that is used is the aqueous solution obtained in the synthesis of the N-oxyl compound.

2. Process according to Claim 1, **characterized in that** N-oxyl compounds added are those from the group of secondary amines in which the N-oxyl group is part of a saturated or unsaturated six-membered ring, and in which each of the C atoms adjacent to the N-oxyl group bears two C1- to C4-alkyl groups.

3. Process according to Claim 1 or 2, **characterized in that** the N-oxyl compound added comprises 2,2,6,6-tetramethylpiperidinyloxyl, 4-hydroxy-2,2,6,6-tetramethylpiperidinyloxyl, 4-oxo-2,2,6,6-tetramethylpiperidinyloxyl and/or 4-ethanoyloxy-2,2,6,6-tetramethylpiperidinyloxyl.

4. Process according to Claim 1 to 3, **characterized in that** the N-oxyl compounds are used in a water/vinyl acetate mixture.

5. Process according to Claim 1 to 4, **characterized in that** 50 to 500 ppm of N-oxyl compound are added per tonne of vinyl acetate in the product gas stream.

6. Process according to Claim 1 to 5, **characterized in that** the N-oxyl compound is added at one point or distributed over two or more points in the plant for working up the product gas stream.

7. Process according to Claim 6, **characterized in that** the N-oxyl compound is added
to the condensate from the preliminary dewatering column and/or
to the gas stream from the preliminary dewatering column and/or
to the return stream to the preliminary dewatering column and/or
to the bottom product from the cycle gas scrubber and/or
to the top product of the azeotrope column and/or
to the return stream into the azeotrope column and/or
to the inlet into the wastewater column and/or
to the return stream into the ethyl acetate column and/or
to the top product of the dewatering column and/or
to the side draw of the dewatering column and/or
to the return stream into the dewatering column and/or
to the top product of the pure vinyl acetate column.

## Revendications

1. Procédé de fabrication d'acétate de vinyle par un procédé en phase gazeuse continu sous catalyse hétérogène, par mise en réaction d'éthylène avec de l'acide acétique et de l'oxygène, puis traitement du courant gazeux de produits, un ou plusieurs composés de N-oxyle, qui contiennent au moins un groupe radicalaire N-oxyle -N-O·, étant ajoutés en tant qu'inhibiteurs pour l'inhibition de la polymérisation de l'acétate de vinyle pendant le traitement du mélange gazeux de produits, **caractérisé en ce que** le composé de N-oxyle est ajouté sous la forme d'une solution aqueuse de 50 à 85 % en poids, la solution aqueuse formée lors de la synthèse du composé de N-oxyle étant utilisée en tant que solution aqueuse du composé de N-oxyle.

2. Procédé selon la revendication 1, **caractérisé en ce qu'**en tant que composés de N-oxyle, des composés du groupe des amines secondaires dans lesquelles le groupe N-oxyle fait partie d'un cycle à six chaînons saturé ou insaturé et dans lesquelles les atomes C voisins du groupe N-oxyle portent chacun deux groupes alkyle en C1 à C4 sont ajoutés.

3. Procédé selon la revendication 1 ou 2, **caractérisé en ce qu'**en tant que composé de N-oxyle, du 2,2,6,6-tétraméthylpipéridinyloxyle, du 4-hydroxy-2,2,6,6-tétraméthylpipéridinyloxyle, du 4-oxo-2,2,6,6-tétraméthylpipéridinyloxyle et/ou du 4-éthanoyloxy-2,2,6,6-tétraméthylpipéridinyloxyle sont ajoutés.

4. Procédé selon les revendications 1 à 3, **caractérisé en ce que** les composés de N-oxyle sont utilisés dans un mélange eau-acétate de vinyle.

5. Procédé selon les revendications 1 à 4, **caractérisé en ce que** 50 à 500 ppm de composé de N-oxyle sont ajoutés par tonne d'acétate de vinyle dans le courant gazeux de produits.

6. Procédé selon les revendications 1 à 5, **caractérisé en ce que** le composé de N-oxyle est ajouté à un emplacement ou réparti sur plusieurs emplacements de l'unité pour le traitement du courant gazeux de produits.

7. Procédé selon la revendication 6, **caractérisé en ce que** le composé de N-oxyle est ajouté au condensat issu de la colonne de pré-déshydratation et/ou au courant gazeux issu de la colonne de pré-déshydratation et/ou au reflux dans la colonne de pré-déshydratation et/ou au produit de fond de l'épurateur de gaz circulaire et/ou au produit de tête de la colonne d'azéotrope et/ou au reflux dans la colonne d'azéotrope et/ou à l'alimentation de la colonne d'eau résiduaire et/ou au reflux dans la colonne d'acétate d'éthyle et/ou au produit de tête de la colonne de déshydratation et/ou au soutirage latéral de la colonne de déshydratation et/ou au reflux dans la colonne de déshydratation et/ou au produit de tête de la colonne d'acétate de vinyle pur.
